# EUROPEAN PATENT APPLICATION

(11) **EP 1 175 931 A1**
(43) Date of publication of application: **30.01.2002**
(21) Application number: 00870169.0
(22) Date of filing: 25.07.2000
(51) Int. Cl.: B01D 15/02, B01D 15/08, B01J 19/10, C07K 1/22, C12M 1/12

(54) **Integration of high cell density bioreactor operation with ultra fast on-line downstream processing**

(71) Applicant: Computer Cell Culture Center S.A., 7180 Seneffe (BE); SMITHKLINE BEECHAM BIOLOGICALS S.A., 1330 Rixensart (BE)
(72) Inventor: Miller, Alain, 7000 Mons (BE)
(74) Representative: Van Malderen, Joelle

(57) **Abstract**

The present invention is related to a recovery and purification method of (a) biological(s) of interest produced by cells retained in a bioreactor under appropriate conditions and by appropriate means for producing the biological(s) inside the bioreactor having a volume higher than 1,5 l culture, and cultivated at a high cell density, preferably at a cell density higher than 10 x 10⁶ cells/ml, said bioreactor being submitted to an acoustic sonoperfusion allowing the recovery of a sonoperfused medium from said bioreactor, characterised in that it is submitted thereafter directly to an Expanded Bed Specific Adsorption for the direct recovery and uninterrupted purification of said biological (5).

The present invention is also related to a process unit for recovering and purifying said biological(s) of interest.

## Description

### Field of the invention

The present invention is related to a process unit based upon the integration of a production of cells grown at high density in a bioreactor with a first step of purification scheme (together referred to as the process) for obtaining and purifying biological products such as (recombinant) (glyco/lipo)proteins, peptides, nucleotides and other 'biologicals' synthesized from said cells growing in said bioreactor.

The present invention is also related to a method for obtaining and purifying said biological(s) from said cells growing in said bioreactor.

### Background of the present invention

Traditionally, growing bacteria or other living cells in the laboratory helps to make biotech products ('biologicals') like active pharmaceutical compounds, nucleic acids, amino acids, vitamins, vaccines, enzymes, membranes, receptors, etc.

Today specific genes of interest are isolated and inserted into living organisms whether pro- or eukaryotic (e.g., plant or animal) cells, which will produce them in large amounts, for instance for the production of new vaccines.

Several problems however hamper the scaling-up from laboratory- to industrial-scale of production of high value quality recombinant biologicals.

Biotechnologists must first have the correct cells and the suitable ingredients to feed them. A system (a bioreactor) has to be designed and built for these cells that can be fed and grown successfully under germ-free conditions. The optimum conditions must be determined to stimulate the cells to produce the maximum amounts of the required quality products as to separate, concentrate and purify(operations commonly referred to as to downstream processing) the sought after products.

However, 'biologicals' especially the ones obtained from mammalian cells cultivated using industrial bioprocesses in operation today, are tremendously expensive to produce. This results from several limitations: low specific productivity and low cell concentration (2 to 3 x 10⁶ cells/ml), use of large culture volume (1000 to 5000 l), large-scale and tedious multi-step downstream processing, costly validated spaces, frequent tear downs (long immobilisation periods), etc.

In addition, prolonged exposure of the highly labile 'biological' to adverse chemical (proteases, pH, etc.) and physical (temperature) conditions during the downstream processing (very large cell culture medium, long time of incubation between successive batch processes, etc.) has also a strong impact on their final quality and costs.

On the other hand, high cell densities (25 to 50 x 10⁶ cells/ml) suitable for the production of recombinant molecules at pilot scale (higher than 1,5 l, preferably higher than 5 l culture) have already been proposed. The cells are retained inside the bioreactor by using acoustic sonoperfusion, allowing therefore the desired biological product to be continuously secreted in the sonoperfusate (US-5,527,460 and US-5,519,051). This system allows the cultivation of cells (bacteria, yeast, animal or plant cells), whether anchorage-dependent or not, at high cell concentration in continuously stirred tank reactor (the configuration most favoured by the biotech industry).

Reduction of fixed costs, increased titers and improved quality (shorter residence time in the bioreactor at 37 °C) of the sought after glycoproteins explain the progressive generalisation of this technology.

The document US-5,711,888 describes a multilayer piezoelectric resonator for the separation of suspended particles. In particular, particles suspended in a fluid can be separated and recycled by means of ultrasonic resonating waves. Preferably, the resonating acoustic field is generated within a multilayer composite resonator system including a transducer, the suspension and a mirror parallel to each other.

Such acoustic resonating-wave-based process is suitable for the separation of all kinds of particles (solid, liquid or gaseous dispersed phases) and is applied for the separation of biological particles such as mammalian (whether anchorage-dependent or not), bacterial and plant cells or aggregates therefrom.

This method includes the use of an acoustic filter to achieve the retention of mammalian cells (whether anchorage-dependent or not) in the bioreactor and/or the selective retention of viable cells relative to non-viable ones.

Until now, prior to downstream processing, the high value/quality biological(s)contained in the feedstock must be freed from the secreting living organisms. This is accomplished by either centrifugation or filtration, methods which are complicated to implement, are time-consuming, expensive to run and introduce a discontinuity in the process between cell growth on the one hand and downstream processing on the other.

### Aims of the invention

The present invention aims to provide a method and process unit that allow to automate and accelerate the recovery and the purification of 'biologicals' obtained from cells, preferably mammalian cells, grown at high cell density in a sonoperfused continuously stirred tank reactor (bioreactor), especially high value 'biologicals' such as membranes, (glyco)proteins or peptides suitable for pharmaceutical, medical or other biochemical processes.

A preferred aim of the present invention is to provide such method and process unit which do not present the drawbacks of the methods and process units of the state of the art and which allow the uninterrupted recovery of 'biological(s)' in high yield, quality and purity.

A further aim of the present invention is to provide such method and process unit which allow the recovery of biological materials at low cost on an industrial scale.

### Summary of the invention

The present invention is related to a process unit combining the technical and economical advantages of a mammalian cell-based bioprocess integrating a high cell density (about 10, but preferably higher than 25 x 10⁶ cells/ml) reactor coupled to an ultrafast (online) downstream purification processing.

In the process unit and method according to the invention, the cells producing the 'biologicals' of interest are retained inside the bioreactor by using an acoustic sonoperfusion system allowing the desired biologicals to be continuously produced in the sonoperfusate, these molecules being thereafter recovered and uninterruptedly purified by direct downstream first purification step.

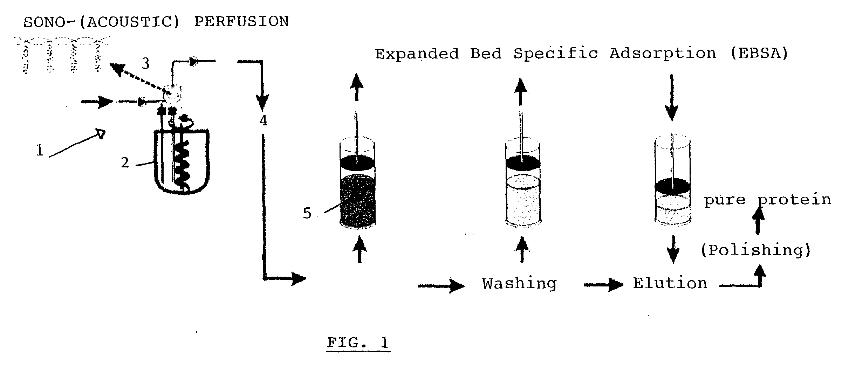

In order to drastically reduce the "biological's" possible chemical (pH, proteolysis, etc.) and physical (temperature) degradation, the sonoperfusate which is almost completely depleted from cells is advantageously directly and uninterruptedly (i.e., without centrifugation and/or filtration) captured on an Expanded Bed Specific Adsorption (EBSA) chromatography column, the support of which is covalently bound to a ligand specifically adsorbing the sought after 'biological' (see Fig. 1).

Therefore, a first aspect of the present invention is related to a recovery and purification method of one or more 'biologicals' of interest produced by cells, preferably mammalian cells (including hybridomas), wherein said cells are retained, preferably in suspension, in a bioreactor by appropriate means for producing the biological product(s) inside the bioreactor having preferably a volume of culture higher than 1,5 l, preferably higher than 5 l culture, at a cell density higher than 10 x 10⁶ cells/ml.

According to the invention, said bioreactor is submitted to an acoustic sonoperfusion (preferably according to the method described in the patent US-5,711,888, incorporated herein by reference), allowing the recovery of a sonoperfusate (harvest medium) from said bioreactor, being submitted thereafter directly to an Expanded Bed Specific Adsorption (EBSA) (preferably upon materials (chromatographic column) described in the patent application WO99/51316, incorporated herein by reference), for the direct recovery and purification of said 'biological(s)', at a purity and recovery rate higher than 75%, preferably higher than 80 or 85%, more preferably higher than 90 or 95% (in weight).

More preferably the Expanded Bed Absorption is performed upon a chromatographic column comprising a dense solid support such as a mineral oxide matrix having a pore volume which is less than 50% of the total volume of the mineral oxide matrix. An interactive polymer network which is rooted in pore and on the surface of the matrix in that said support comprises a specific ligand of the product will be recovered and purified.

It is meant by "(a) biological(s) of interest", any macromolecule or group of macromolecules of pharmaceutical, medical, or biochemical interest, produced by cell(s), such as viruses, receptors, vaccines, enzymes, nucleic acids or membranes, made of proteinic, glycoproteinic, peptidic, polysaccharidic and/or lipidic structure, including antibodies and hypervariable portions thereof.

Preferably, said biological products are recombinant molecules obtained from recombinant microorganisms, preferably from recombinant mammalian cells (whether anchorage-dependent or not) or hybridomas.

It is meant by "the recovery and the purification of (a) biological(s)", the specific isolation (on an industrial scale) of said biological(s) from possible living or soluble contaminants such as cells or cell debris.

It is meant by an "uninterrupted" purification and direct recovery upon an Expanded Bed Specific Adsorption, the fact that in the process unit and in the method according to the invention, the sonoperfusate exiting from the bioreactor does not undergo nor a centrifugation nor a filtration before being fed to the EBSA column for the recovery and the purification of the biological(s) of interest.

It is also possible to combine with the method according to the invention, other purification steps for obtaining the biological product of interest highly pure in liquid or solid form.

The present invention is also related to a process unit and a plant comprising several process units with the means for performing the method according to the invention, especially a bioreactor (higher than 1,5 l culture bioreactor), comprising means for integrating a high cell density (higher than 10 x 10⁶ cells/ml, preferably higher than 25 x 10⁶ cells/ml) combined with an acoustic sonoperfusion system and means for the recovering of the sonoperfused medium (harvest medium) from said bioreactor, coupled directly to an Expanded Bed Specific Adsorption means (chromatography column) for the recovery and the purification of 'biological(s)' of interest produced by said cells and present in said medium.

It is meant by a process unit the combination of several devices which allow preferably continuously the culture of cells and the downstream purification of products obtained from said cells which can be thereafter directly used for their therapeutic or biochemical properties in a highly pure form.

It is meant by a plant comprising several process units the possibility to comprise in a single operation system or factory several of said process units working preferably in parallel for the simultaneous production of various identical or different biologicals according to the GMP requirements.

In the method and process unit according to the invention, the Expanded Bed Specific Adsorption chromatography column comprises specific ligands resistant to the activity of proteases, which are possibly present in the medium.

Advantageously, said chromatography column is an affinity chromatographic column comprising macroporous microbeads made of a matrix such as zircon oxide or quartz characterised by a high porosity, a high physical rigidity, a high specific gravity (with a final density higher than 1.4 g/cm³), an important flow rate (in the range of 500 to 1000 cm³/h), a high mass transfer, a strong chemical stability, able to resist to sanitisation for 2 hours with at least 0.5N NaOH and a high specific binding capacity. Said specific ligands coupled to said beads, are resistant to the activity of possible proteases or other enzymatic molecules present in the sonoperfused medium and, are preferably selected by combinatorial or aptamer chemistry.

The combination of a bioreactor with uninterrupted purification allows advantageously the production of biologicals of interest in high yield and purity and the industrial automation of the production and purification of these high value biologicals.

### Short description of the drawings

Fig. 1 represents an approach for the production and purification of recombinant molecules according to the invention.

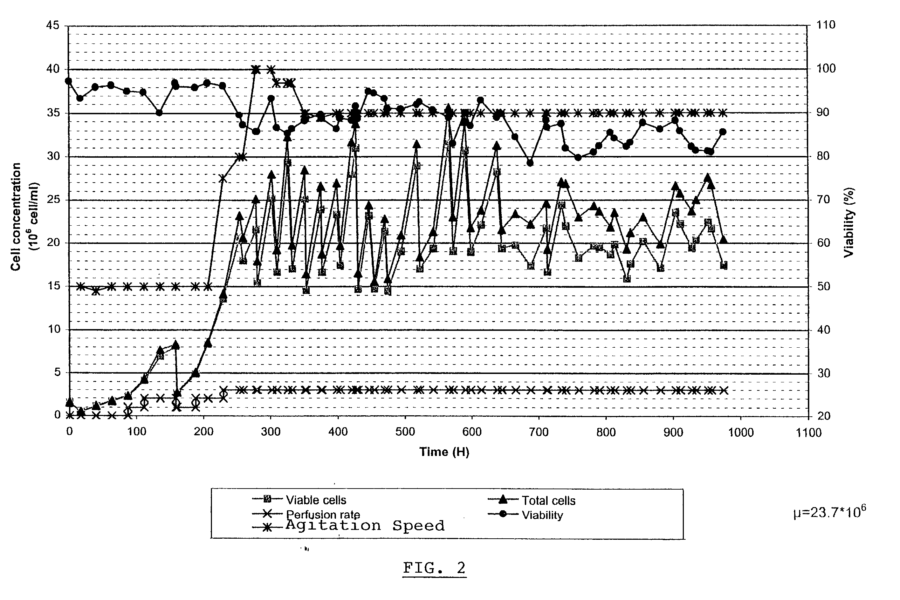

Fig. 2 represents the long term (4 weeks) sonoperfused culture of recombinant CHO-K1 cells secreting a recombinant truncated gD protein of an Herpes virus HSV-2 such as described in the patent EP 0 139 417 B2.

Fig. 3a and 3b represent analyses confirming the stability of recombinant protein gD isolated during the plateau cultivation at 25 X 10⁶ cells/ml (day 0, day 5, day 10, day 15, day 20, day 25 and day 30) (SDS-PAGE/Coomassie staining and lectin analysis).

### Detailed description of the invention

As shown in Fig. 1, the almost quantitative retention of the cells inside a bioreactor 2 of the process unit 1 according to the invention is obtained by acoustic filtration means 3 that allow the recovery of a resulting almost cell-free perfusate (harvest medium) 4 that could be submitted directly in a straightforward way to fractionation by chromatography by using Expanded Bed Specific Adsorption means 5 with zircon oxide or other high dense support.

By the method and process unit according to the invention, it is possible to obtain the recovery of biological molecules such as (glyco/lipo)proteins with a high purity.

With the present invention, pitfalls resulting from the method of the state of the art are completely eliminated, giving rise to a process which integrates in a single operation cell growth and downstream plant or process unit, a direct and single-step clarification, concentration and initial fractionation at a high yield and purity, this at low costs.

As expected, the biologicals of interest (recovered and purified) maintain their integrity and can be used for their advantageous therapeutic or industrial properties.

Furthermore, by using specific ligands in the zircon oxide-based Expanded Bed Specific Adsorption according to the invention, it is possible to solve the drawbacks of the state of the art such as degradation due to the presence of proteases in the harvest medium.

The present invention will be described in more details in the following non-limiting examples.

### Example 1

The process unit according to the invention comprises a 8L culture volume bioreactor and two acoustic sorting flow cells working synergistically (obtained from Anton Paar GmbH, Austria) for the growth of a CHO-K1 cell line. The 8 l culture volume bioreactor is adapted for high density cell culture in sonoperfusion (up to 25 x 10⁶ cells/ml). Said cell concentration could be maintained during a long period (for at least four weeks).

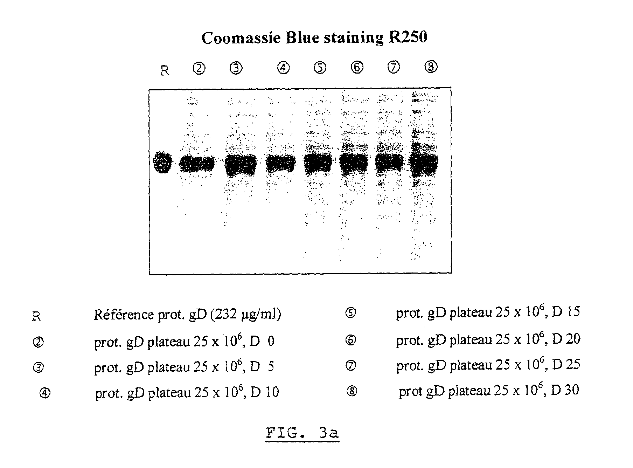

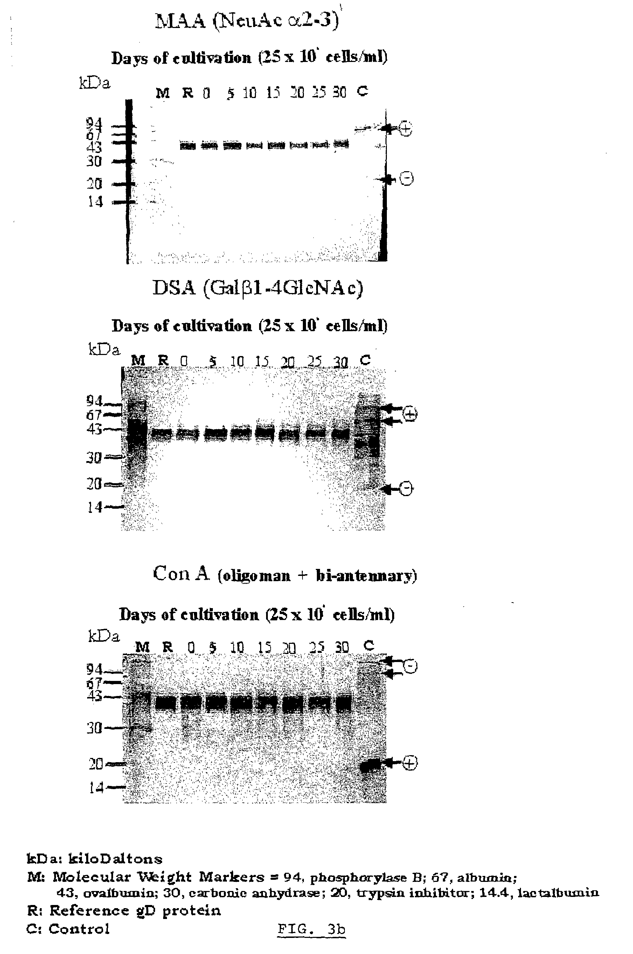

The CHO K1 cell line was selected for its ability to produce a recombinant truncated gD protein of an Herpes virus HSV-2 such as described in the patent EP 0 139 417 B2. The recombinant protein produced at a high cell concentration (15 to 33 X 10⁶ cells/ml) exhibits the same biochemical characteristics as a reference expressed and purified by classical techniques (see Fig. 3a and 3b, and table 2).

The harvest obtained from the bioreactor according to the invention was submitted to an Expanded Bed Specific Adsorption purification made out of a column with zircon oxide beads bound to a ligand selected from a dye library. Said ligand has been immobilised upon the agarose or another gel incorporated in said beads.

Taking into account binding and elution capacity (elution yield and protein gD purity in eluates), the specific dye according to the invention has been selected as the most suitable specific dye for the protein gD from cell culture contaminants (cells and cell debris, proteins secreted in the medium, etc.).

Other ligands, especially other dyes, could be selected by the person skilled in the art according to routine experiments based upon the affinity constant and the capacity value of each ligand to recover the biological molecule of interest.

The various parameters and the characteristics for the recovering of proteins are presented in the following table 1.

**Table 1**

| | |
|---|---|
| Volume of perfusate | 40 l |
| Volume of packed specific ligand bound zircon oxide particles | 0.2 l |
| Volume of elution (3x the volume of packed specific ligand bound support) | 0.6 l |
| Total capacity | 4.6 mg recombinant protein / ml specific ligand bound on zircon oxide support |
| Loading time | 6 l/h |
| Eluted recombinant protein purity | ≥ 85% (SDS-PAGE/ 1-D densitometry) |
| Eluted recombinant protein recovery | ≥ 85% (ELISA) |

The characteristics of said protein (molecular weight, 3D conformation, chromatogram, etc.) have shown that the recovered glycoprotein has maintained its integrity, in the recovery and purification method and plant (process unit) according to the invention.

Quality control of protein gD produced and purified during the whole cultivation process (4 weeks) has been confirmed by studying the glycosylation status of samples isolated during the plateau cultivation at 25 x 10⁶ cells/ml (day 0, day 5, day 10, day 15, day 20, day 25 and day 30).

**Table 2**

| Time of plateau cultivation (D = day) | prot tot harvest (*µ*g/ml) | GD protection harvest (*µ*g/ml) | specific productivity *µ*g/10⁶ cell |
|---|---|---|---|
| D 0 | 217 | 48 | 6,6 |
| D 5 | 280 | 60 | 6,8 |
| D10 | 253 | 35 | 5,1 |
| D15 | 262 | 52 | 6,5 |
| D20 | 292 | 48 | 5,3 |
| D25 | 282 | 37 | 4,9 |
| D30 | 316 | 55 | 8,0 |
| **Average** | 272 | 48 | 6 |
| **Standard dev.** | 31 | 9 | 1 |

These purified samples have confirmed a totally similar glycosylation status by their monosaccharide composition, identical lectin detection (see Fig. 3b), and by the characterization of the N-glycan chains (MALDI-TOF-MS).

### Example 2

### Production of monoclonal antibodies (IgG) secreted from a hybridoma cell line cultivated at high densities and purification by EBSA with the MEP (4-Mercapto-Ethyl-Pyridine) ligand immobilised on zirconia oxide beads

A murine hybridoma cell line is cultivated in the presence of DMEM medium supplemented with 2% Foetal Calf Serum (FCS).

Hybridoma cells for the Master Cell Bank have been grown in Techne glass spinner vessels until 160-ml volume at a concentration of 1.6 X 10⁶ cells/ml, providing 25 cryotubes of 10⁷ cells/tube.

The Working Cell Bank was established from fresh stocks of hybridoma cells thawed from an ampoule in the presence of DMEM supplemented with 10% FCS.

Several assays for preparing these WCBs have been performed by freezing cells cultivated at high density (10 X 10⁶ cells/ml) in ampoules.

Two successive inoculations using cells grown at 10 x 10⁶ cells/ml confirmed these results in 1.5-l bioreactors (validation of the viability process).

It was initially decided to progressively increase the cell concentration (at an 1.5-l scale) until 10 x 10⁶ cells/ml and, in a second phase, to maintain this cell concentration during a long period (4 weeks).

Culture sonoperfusate ('waste') has been collected at various cell concentrations (2.5, 5, 7.5, and 10 x 10⁶ cells/ml) in order to start downstream purification at pilot scale. The secreted IgG protein present in these medium batches was then dispatched in order to be further characterised and compared with the reference monoclonal antibody IgG (produced at cell density of 1-2 x 10⁶ cells/ml for 2-4 days and purified by 'classical' chromatography with Protein A adsorbent).

The first cultivations at 8-10 x 10⁶ cells/ml could be maintained at this high density for 2-3 weeks without experiencing a dramatic decrease in cell viability. In addition to cell cultivation improvements, additional controls are now performed on line in the bioreactors (determination of LDH and glucose concentrations).

The ligand chosen for a IgG specific binding is 4-Mercapto-Ethyl-Pyridine (MEP). In contrast to protein A sorbents, IgG binding is essentially independent of subclass or species, is protease-resistant and chemically stable to long term cleaning with 1 M sodium hydroxide.

The MEP is immobilised on high density zirconia matrix following protocols previously described.

The monoclonal antibodies were directly captured from undiluted sonoperfusate by using this MEP-zirconia adsorbent. The sonoperfusate containing the secreted antibodies was applied on the EBSA resin without adjustment (neutral pH, independence of ionic strength).

After an extensive washing with PBS, a gentle elution of the antibody (reducing aggregate formation) was obtained by simply reducing the pH to 4, at low ionic strength (elution buffer: 50 mM sodium acetate, pH 4).

In a first step, the binding and elution conditions have been optimised by using a column specifically dedicated to analytical experiments on fluidised bed (FastLine 10, Upfront, Denmark).

Breakthrough curves for the ligand (immobilised on 5 ml of zirconia beads) were determined by loading relatively large volumes (400-500 ml) of sonoperfused mediums collected from three hybridoma cell cultures (grown at around 10 x 10⁶ cells/ml). Western blot and ELISA analyses clearly demonstrated that MEP ligand exhibits a high IgG binding capacity (full capacity of the resin has not been reached under these conditions).

Dynamic binding capacity was determined by estimating the concentration of IgG eluted from these three hybridoma supernatants. SDS-PAGE (Coomassie and silver nitrate stainings), Western blot and ELISA analyses unanimously confirmed that MEP is the best candidate for capturing IgG from the cell supernatant: a capacity above 10 mg protein IgG/ml resin and a purity exceeding 90 % have been repetitively obtained for this ligand

The second step of the EBSA downstream processing focused on the optimisation of the elution conditions.

SDS-PAGE, Western blot, ELISA and FPLC analyses unambiguously showed that no apparent difference has been detected for the antibody binding capacity and purity.

In a third step, the EBSA downstream processing has been scaled-up to larger volumes by using a Streamline 50 column (Amersham Pharmacia).

First, a 200-ml batch of resin (with immobilised MEP) was controlled in the analytical FastLine 10 column: a sample (5 ml) exhibited similar properties (density, dynamic binding capacity, breakthrough curves, etc.) than the zirconia beads used for the selection of the best capacitive ligands.

Thereafter, large volumes of hybridoma culture mediums (grown at 10 x 10⁶ cells/ml) were loaded on a Streamline 50 column containing 200 ml of MEP-zirconia oxide beads. Western blot and ELISA analyses indicated that the secreted protein K is completely captured from 2 litres, and even 8 litres, of cell supernatants (more than 200 mg of IgG with a purity higher than 90 %).

## Claims

1. Recovery and purification method of a biological of interest produced by cells retained in a bioreactor under appropriate conditions and by appropriate means for producing the biological(s) inside the bioreactor having a volume higher than 1,5 l culture at a high cell density, preferably at a cell density higher than 10 x 10⁶ cells/ml, said bioreactor being submitted to an acoustic sonoperfusion allowing the recovery of a sonoperfused medium from said bioreactor, **characterised in that** it is submitted thereafter directly to an Expanded Bed Specific Adsorption for the direct recovery and purification of said biological.

2. Method according to the claim 1, **characterised in that** the cells are mammalian cells (whether anchorage-dependent or not), including hybridomas.

3. Method according to the claim 1 or 2, **characterised in that** the biological is a macromolecule or a group of macromolecules being an active pharmaceutical compound or an enzyme.

4. Method according to any one of the preceding claims, **characterised in that** the Expanded Bed Specific Adsorption is performed upon a chromatography column comprising dense solid support, such as a mineral oxide matrix, having a pore volume which is less than 50% of the total volume of the mineral oxide matrix, and an interactive polymer network which is rooted in pores and on the surface of the matrix and **in that** said support comprises specific ligands to the biological(s) of interest to be recovered and purified.

5. Method according to the claim 4, **characterised in that** the ligand is protease-resistant and specific to the biological of interest to be purified and recovered.

6. Process unit (1) for the recovery and the purification of a biological of interest, **characterised in that** it comprises a bioreactor (2) having a volume higher than 1,5 l culture comprising means for integrating a high cell density, preferably a density higher than 10 x 10⁶ cells/ml, inside the bioreactor, combined with an acoustic sonoperfusion system and means (3) for the recovery of a sonoperfused medium (4) from said bioreactor (2), coupled directly to an Expanded Bed Specific Adsorption means (5) for the recovery and the purification of the biological(s) of interest produced by said cells and present in said medium.

7. Process unit according to the claim 6, **characterised in that** the cells are mammalian cells (whether anchorage-dependent or not) including hybridomas.

8. Process unit according to the claim 6, **characterised in that** the cells are other eukaryotic cells (yeast, insect cells, etc.) or prokaryotic cells (bacteria, etc.).

9. Process unit according to the claims 6 to 8, **characterised in that** the biological is a macromolecule or a group of macromolecules being an active pharmaceutical compound or an enzyme.

10. Process unit according to any one of the claims 6 to 9, **characterised in that** the Expanded Bed Specific Adsorption means are dense solid supports, such as mineral oxide, integrated in a chromatography column having a mineral oxide matrix with a pore volume which is less than 50% of the total volume of the highly dense matrix, and an interactive polymer network which is rooted in pores and on the surface of the mineral oxide matrix and **in that** said support comprises specific ligands to the biological(s) of interest to be recovered and purified.

11. Process unit according to the claim 10, **characterised in that** the ligand is protease-resistant, sanitisable, and has been selected to be highly specific for the biological(s) of interest.
